# EUROPEAN PATENT SPECIFICATION

(11) **EP 2 594 938 B1**
(45) Date of publication and mention of the grant of the patent: **26.08.2015**
(21) Application number: 11806343.7
(22) Date of filing: 11.07.2011
(51) Int. Cl.: G01N 33/48, G01N 33/68

(54) **EX-VIVO METHOD FOR THE EARLY DIAGNOSIS OF MINIMAL HEPATIC ENCEPHALOPATHY BY MEANS OF THE DETERMINATION OF 3-NITROTYROSINE IN SERUM**
EX-VIVO-VERFAHREN ZUR FRÜHERKENNUNG MINIMALER HEPATISCHER ENZEPHALOPATHIE DURCH NACHWEIS VON 3-NITROTYROSIN IM SERUM
MÉTHODE EX VIVO POUR LE DIAGNOSTIC PRÉCOCE DE L'ENCÉPHALOPATHIE HÉPATIQUE MINIME PAR DÉTERMINATION DE LA PRÉSENCE DE 3-NITROTIROSINE DANS LE SÉRUM

(30) Priority: 12.07.2010 ES 201000899
(43) Date of publication of application: 22.05.2013
(73) Proprietor: Fundación De La Comunidad Valenciana Centro De Investigacion Principe Felipe, 46012 Valencia (ES); Fundación Para la Investigación Biomédica, la Docencia y la Cooperación Internacional y el De Desarrollo del Hosp. Clínico Univ. de Valencia, 46010 Valencia (ES)
(72) Inventor: FELIPO ORTS, Vicente, E-46012 Valencia (ES); CAULÍ, Omar, E-46012 Valencia (ES); MONTOLIU FÉLIX, María del Carmen, E-46010 Valencia (ES)
(74) Representative: Carlos Hernando, Borja
(86) International application number: PCT/ES2011/070509
(87) International publication number: WO 2012/007624

(56) References cited:
- MONTOLIU ET AL: "IL-6 and IL-18 in Blood May Discriminate Cirrhotic Patients With and Without Minimal Hepatic Encephalopathy", JOURNAL OF CLINICAL GASTROENTEROLOGY, RAVEN PRESS LTD., NEW YORK, NY, US, vol. 43, no. 3, 1 March 2009 (2009-03-01), pages 272-279, XP008167974, ISSN: 0192-0790
- ISABEL SUÁREZ ET AL: "Induction of NOS and nitrotyrosine expression in the rat striatum following experimental hepatic encephalopathy", METABOLIC BRAIN DISEASE, KLUWER ACADEMIC PUBLISHERS-PLENUM PUBLISHERS, NE, vol. 24, no. 3, 18 September 2009 (2009-09-18), pages 395-408, XP019751976, ISSN: 1573-7365, DOI: 10.1007/S11011-009-9154-5
- GORG B ET AL: "Inflammatory cytokines induce protein tyrosine nitration in rat astrocytes", ARCHIVES OF BIOCHEMISTRY AND BIOPHYSICS, ACADEMIC PRESS, US, vol. 449, no. 1-2, 15 May 2006 (2006-05-15), pages 104-114, XP024943182, ISSN: 0003-9861, DOI: 10.1016/J.ABB.2006.02.012 [retrieved on 2006-05-15]
- CARMINA MONTOLIU ET AL: "3-Nitro-Tyrosine as a Peripheral Biomarker of Minimal Hepatic Encephalopathy in Patients With Liver Cirrhosis", THE AMERICAN JOURNAL OF GASTROENTEROLOGY, vol. 106, no. 9, 12 April 2011 (2011-04-12), pages 1629-1637, XP055073367, ISSN: 0002-9270, DOI: 10.1038/ajg.2011.123
- MONTOLIU C. ET AL.: '3-Nitro-Tyrosine as a Peripheral Biomarker of Minimal Hepatic Encephalopathy in Patients With Liver Cirrhosis.' THE AMERICAN JOURNAL OF GASTROENTEROLOGY vol. 106, no. 9, 12 April 2011, pages 1629 - 1637, XP055073367
- SUAREZ I. ET AL.: 'Induction of NOS and nitrotyrosine expression in the rat striatum following experimental hepatic encephalopathy.' METABOLIC BRAIN DISEASE vol. 24, no. 3, 18 September 2009, pages 395 - 408, XP019751976
- MARTIN D. ET AL.: 'Oxidative stress and hippocampus in a low-grade hepatic encephalopathy model: protective effects of curcumine.' HEPATOLOGY RESEARCH: THE OFFICIAL JOURNAL OF THE JAPAN SOCIETY OF HEPATOLOGY. vol. 38, no. 11, 2008, pages 1148 - 1153, XP055073368
- GORG B. ET AL.: 'Inflammatory cytokines induce protein tyrosine nitration in rat astrocytes.' ARCHIVES OF BIOCHEMISTRY AND BIOPHYSICS vol. 449, no. 1-2, 09 March 2006, pages 104 - 114, XP024943182
- ODEH M ET AL.: 'Serum levels of tumor necrosis factor-a correlate with severity of hepatic encephalopathy due to chronic liver failure.' LIVER INTERNATIONAL vol. 24, no. 2, April 2004, pages 110 - 116, XP055073370
- MONTOLIU, C. ET AL.: 'IL-6 and IL-18 in Blood May Discriminate Cirrhotic Patients With and Without Minimal Hepatic Encephalopathy.' JOURNAL OF CLINICAL GASTROENTEROLOGY. vol. 43, no. 3, March 2009, pages 272 - 279, XP008167974
- BEMEUR C. ET AL.: 'Evidence for oxidative/nitrosative stress in the pathogenesis of hepatic encephalopathy.' METABOLIC BRAIN DISEASE vol. 25, no. 1, March 2010, pages 3 - 9, XP019793102
- CALABRESE V. ET AL.: 'Nitrosative Stress, Cellular Stress Response, and Thiol Homeostasis in Patients with Alzheimer's Disease.' ANTIOXIDANTS AND REDOX SIGNALING vol. 8, no. 11-12, November 2006, pages 1975 - 1986, XP055073373
- RABBANNI, N. ET AL.: 'Assay of 3-Nitrotirosin in Tissues and Body Fluids by Liquid Chromatography with Tandem Mass Spectrometric Detection.' METHODS IN ENZYMOLOGY vol. 440, 2008, pages 337 - 359, XP008167973

## Description

### Technical Field of the Invention

The present invention is included in the field of pharmacology and medicinal chemistry and relates to an *ex-vivo* method for the detection and early diagnosis of minimal hepatic encephalopathy (MHE) in patients with liver diseases, including cirrhosis, based on the presence of specific serum biomarkers, specifically by means of determining 3-nitrotyrosine (3-NT).

### Background of the Invention

Hepatic encephalopathy (HE) is a medical term assigned to describe a neuropsychiatric abnormality caused by prior alteration of liver function. Hepatic encephalopathy can be a progressive and chronic disorder or a disorder of acute onset and is reversible in some cases. HE is particularly common in cirrhoses. The term minimal hepatic encephalopathy (MHE) refers to subtle changes in cognitive function and electroencephalographic patterns in patients with liver diseases (including cirrhosis) that show no clinical evidence of hepatic encephalopathy.

This liver disease complication generally goes unnoticed by doctors. Between 30% and 50% of liver cirrhosis patients that do not show evident hepatic encephalopathy (HE) symptoms have MHE with slight cognitive impairment. MHE is not detected in routine analysis but can be detected using psychometric tests or by performing neurophysiological studies (1 -4).

MHE is the first phase of the HE spectrum (5, 6) and is associated with a reduction in the patients' quality of life and in their ability to perform everyday tasks, including driving vehicles (7, 8). MHE increases the risk of suffering occupational and traffic accidents (9), predicts the onset of clinical HE (10) and is associated with a lower survival (11).

The early detection of MHE would allow treating the patients and would improve their quality of life, prevent or delay the onset of clinical HE and increase patient survival. It is therefore necessary to have simple methods for diagnosing MHE in patients with liver diseases.

In the state of the art, Hernandez-Avila *et al.* 2001 (12) review different diagnostic markers and neurochemical mechanisms of encephalopathy due to liver failure. The document indicates how hepatic encephalopathy (HE) is of a multifactorial origin and that it results from the liver's inability to remove substances with neuromodulating properties from plasma. Specifically, in the section dedicated to the ammonium hypothesis, the document shows how it is known that an increase in blood ammonium levels results in greater ammonium uptake in the brain, generating the neurotoxic effect.

Marco Sezolo *et al.* 2004 (13) diagnose MHE by means of neuropsychological and neurophysiological tests in liver cirrhosis patients and compare the different methods of diagnosis (PSE, partial NH₃ pressure [pNH₃] and neurophysiological tests) to evaluate their efficiency with respect to hepatic encephalopathy diagnosis.

Keiding *et al.* 2006 (14) study the brain metabolism of ammonium (¹³N-Ammonia) by means of positron emission tomography (PET) in cirrhosis and acute hepatic encephalopathy (HE) patients, in non-hepatic encephalopathy cirrhosis patients and in healthy patients. From the studies performed it is concluded that cirrhosis and acute hepatic encephalopathy patients show an increase in brain ammonium uptake mainly due to an increase in blood ammonia levels and not so much due to a change in brain ammonia kinetics.

Suarez *et al.* 2006 (15) attempt to determine if astroglia and neuron alterations are mediated by nitric oxide (NO) in experimental HE models (caused by a portacaval [PCA] shunt). Specifically, the expression of NO synthases (nNOS and iNOS) and protein tyrosine nitrosylation in the corpus striatum of rats exposed to PCA for 1 month and 6 months were studied. The results of the research show how in the animal model of hepatic encephalopathy the iNOS levels and protein tyrosine nitrosylation gradually increase in astrocytes as the exposure to PCA increases.

Görg *et al.* 2006 (16) study the effects of inflammatory cytokines on tyrosine nitration in cultured rat astrocytes and in the brains of live rats and concludes that it may be important for these and for the pathogenesis of HE and other diseases which involve exposing the brain to cytokines.

Rabbani, N. and Thornalley, P.J. 2008 (17) describe an assay for determining free 3-NT in biological fluids and tissues by means of liquid chromatography together with mass spectrometric detection. The results of 3-NT obtained in patients with diabetes, cirrhosis, chronic and acute renal failure, and neurological diseases, including Alzheimer's disease, obtained using their method and other independent measurements are compared.

More recently, Bragagnolo Jr M A *et al.* 2009 (18) show that MHE consists of a set of neuropsychiatric alterations in individuals with liver failure and/or portal hypertension. The physiopathology thereof is complex and very likely multifactorial even though ammonium levels seem to best explain the neuropathological and clinical alterations observed. From reading the document, it is concluded that arterial blood ammonium concentration does not play a significant role in diagnosing MHE.

Today, MHE can only be diagnosed by means of neuropsychological and neurophysiological tests (electroencephalogram). The early diagnosis of MHE can have prognostic and therapeutic implications in cirrhosis patients. Therefore, it would be very convenient and useful in clinical practice to have a peripheral biomarker which could be measured *ex-vivo* in the patient's blood and which reflects the presence of MHE in patients with liver diseases, such as liver cirrhosis.

### Object of the Invention

The present invention relates to a method of diagnosis performed *ex-vivo* in cirrhosis patients which allows determining the presence of MHE through the detection of a specific biomarker such as 3-NT, thus contributing to the early treatment thereof and improving the quality of life of these patients.

The current reference method for detecting MHE is a battery of 5 psychometric tests known as "Psychometric Hepatic Encephalopathy Score" (PHES) (2). However, this method is not routinely used in clinical practice because it is time consuming and requires adjustments depending on the age and education level of individuals.

The authors of the present application have demonstrated that the presence of MHE is correlated with an increase in the activation of soluble guanylate cyclase by nitric oxide in lymphocytes (3) and that the serum pro-inflammatory cytokine, specifically IL-6 and IL-18, levels also correlate with MHE (4). However, these parameters are not being used in diagnosing MHE. For that reason, other serum biomarkers which can detect MHE with a better predictive value have been sought.

Studies in animal models of HE have shown that some factors contributing to cognitive impairment are cyclic GMP and nitric oxide alterations in the brain (19, 20) and neuroinflammation (21).

### Detailed Description of the Invention

The *ex-vivo* method for the early diagnosis of minimal hepatic encephalopathy (MHE) in liver cirrhosis patients according to the present invention comprises the following steps:
a) obtaining serum or plasma obtained from the blood of patients and controls, and
b) determining whether or not the measurement of the concentration of 3-nitrotyrosine obtained in (a) is greater than a specific level.

To that end, a series of parameters, including cyclic GMP, nitrite/nitrate (stable nitric oxide metabolites), and 3-NT (formed from peroxynitrite, a metabolite that is more toxic than nitric oxide) levels, has been analyzed in the serum of 43 control subjects without liver disease; 44 non-MHE cirrhotic patients and 47 MHE patients. Since liver failure alters amino acid metabolism, certain amino acid levels have also been determined. It has been analyzed if any of the parameters determined is useful for diagnosing the presence of MHE. The sensitivity, specificity and positive and negative predictive values for each parameter have been analyzed as an indicator of the presence of MHE evaluated by means of the PHES battery of psychometric tests.

### Subjects and Methods

### Patients and controls

"Patients" are subjects diagnosed with liver cirrhosis after histological study and "controls" are subjects in which the presence of liver disease has been ruled out. A study has been performed with patients and with control subjects, i.e., a study has been performed with patients diagnosed with liver cirrhosis after histological study and with control subjects in which the presence of liver disease has been ruled out. None of the patients was under antibiotic treatment nor had any patient suffered previously from bacterial peritonitis or TIPS (transjugular intrahepatic portosystemic shunt).

After a physical examination blood was drawn for the determination of routine biochemical parameters and for the determinations described below. The study protocol complies with the ethical standards of Declaration of Helsinki, 1975 (22) and was approved by the hospital Ethics and Scientific Committees.

After performing the battery of psychometric tests, the patients were classified as non-MHE and MHE patients (see below). Therefore, the study includes three groups: 1) control subjects, 2) non-MHE patients, and 3) MHE patients.

The composition of the groups, age and the liver disease etiology are indicated in Table 1.

Serum was drawn from 43 controls, 44 non-MHE patients and 47 MHE patients for determining the selected substances (see below). The number of samples analyzed for each compound is given in Table 2.

### Detection of the presence of MHE using the PHES battery of psychometric tests

The presence of MHE was determined using the PHES battery recommended as the reference method for detecting MHE (2, 16, 17). The score was calculated by adjusting it depending on age and education level using the normality tables for the Spanish population available at www.redeh.org.

Those patients with a score of -4 or less (23, 24) were considered to have MHE.

### Obtaining serum

Five milliliter of blood were drawn in BD Vacutainer tubes and centrifuged at 500 g for 10 minutes. The supernatant was collected and kept at -80°C in aliquots of 500 µL.

### Activation of soluble quanylate cyclase by the nitric oxide generating agent, S-nitroso-N-acetyl penicillamine (SNAP), in intact lymphocytes

The lymphocytes were prepared as described by Kimura *et al.* (25) and the activation of the guanylate cyclase was analyzed as described in (26). Cyclic GMP (cGMP) was determined using the Amersham BIOTRAK cGMP enzyme immunoassay kit (GE Healthcare, Life Sciences, UK).

### Determination of serum amino acids and 3-nitrotyrosine

One hundred microliters of a cold methanol/chloroform (1:2) mixture were mixed with 50 µL of serum and centrifuged at 4°C for 10 minutes. The supernatants were collected and frozen at -80°C.

The concentrations of amino acids and 3-nitrotyrosine were measured in 50 µL of supernatant by HPLC using a reversed-phase HPLC system with o-phthalaldehyde precolumn derivatization and detection by fluorescence as described in (27).

### Statistical analysis

The results were analyzed by means of one-way ANOVA followed by post-hoc Newman-Keuls test using the GraphPad PRISM software Version 3.0. The level of probability accepted as significant is p <0.05.

The upper limit of normality of each variable was calculated according to the usual criterion, considering the mean ± 2 times the standard deviation of the control values. This value was considered as the cut-off point in the sensitivity, specificity and positive and negative predictive value analyses of the different parameters. For these calculations, the data was processed with the SPSS software version 17.0 (SPSS Inc, Chicago, USA).

### Results

As was already known, the patients showed an altered pattern of blood amino acids (Table 2) with a significant increase of aromatic amino acids, tyrosine and phenylalanine, a notable increase (more than 2 times) of methionine, homocysteine and homoserine, and slight increases of asparagine, citrulline, isoleucine and alanine.

Branched amino acid, specifically leucine and valine, levels and glutamate levels are significantly reduced (Table 2).

When comparing amino acid levels in non-MHE and MHE patients, slight differences in glutamine, homocysteine and isoleucine are observed. The differences are more notable for citrulline and particularly for methionine.

In non-MHE cirrhotic patients, citrulline increases to 161±17% with respect to the control subjects, reaching 29±3 µM (Table 2). Citrulline increases significantly (p=0.002) in MHE patients compared with non-MHE patients, reaching 50±5 µM (172±17% with respect to the non-MHE patients and 278±28% with respect to the controls). In non-MHE patients, methionine increases to 232±19% with respect to the controls, reaching 44±3 µM (Table 2). Methionine increases significantly (p<0.0002) in MHE patients compared with non-MHE patients, reaching 73±7 µM (166±16% with respect to the non-MHE patients and 384±36% with respect to the controls).

The increase in 3-nitrotyrosine is particularly notable. The serum concentration of this tyrosine derivative is very low in control subjects (3.3±2.2 nM), in non-MHE patients it increases significantly (p<0.01) to 6.0±4.2 nM, and in MHE patients it greatly increases, reaching 48±24 nM. This represents an 8-fold increase with respect to the non-MHE patients and a 15-fold increase with respect to the controls (Table 2).

Parameters relating with cGMP homeostasis were also determined in the same individuals. The levels are given in Table 3.

### Analysis of the diagnostic usefulness of the determined parameters

The diagnostic usefulness of each of the determined parameters for discriminating MHE patients from non-MHE patients has been analyzed. The results of the PHES battery of psychometric tests have been used as a criterion to define which patients have MHE and which do not. Patients obtaining a result equal to or less than - 4 were considered as having MHE.

The following parameters were determined:
Sensitivity: the proportion of MHE patients correctly identified as such.
Specificity: the proportion of non-MHE patients correctly identified.
Positive predictive value: the proportion of patients with a positive test result that are correctly diagnosed.
Negative predictive value: the proportion of patients with a negative test result that are correctly diagnosed.

The values of said parameters are given for each substance analyzed in Table 4.

The results show that 3-nitrotyrosine is a good indicator of the presence of MHE in patients, showing good sensitivity (82%) and specificity (71%) and positive and negative predictive values of 75% and 79%, respectively (Table 4).

Methionine also shows good sensitivity (82%) but less specificity (56%) and positive and negative predictive values of 65% and 75%, respectively (Table 4).

When analyses are performed by combining two substances, the specificity and the positive predictive value increase but sensitivity decreases (Table 4).

### Discussion

3-NT is formed from peroxynitrite, a nitric oxide metabolite. It is well known that nitric oxide increases in the blood of liver cirrhosis patients. However, this increase is similar in the MHE and non-MHE patients (3,4). This is confirmed in the present study. The levels of nitrite + nitrate, stable nitric oxide metabolites, are not statistically different in MHE and non-MHE patients (Table 3).

Therefore it is impossible to predict from the data in the literature that 3-nitrotyrosine (or any other nitric oxide metabolite) could be a good indicator of the presence of MHE.

It has been found that blood 3-NT levels increase significantly in cirrhotic MHE patients compared with the levels in non-MHE patients and that serum 3-NT levels have a predictive value for predicting the presence of MHE in patients (Table 4).

The mechanisms leading to this alteration in 3-NT levels in MHE patients is currently unknown.

Until now no biomarker which can measure the presence of MHE *ex-vivo* in blood samples has been described. MHE is detected only by means of psychometric or neurophysiological tests.

Although the present group has found correlations between the activation of soluble guanylate cyclase by nitric oxide (3) and IL-6 and IL-18 levels (4) with the degree of MHE, these parameters are not used for diagnosing MHE as they do not have suitable predictive, sensitivity and specificity values. The data of the present application show that 3-NT has a greater predictive value for diagnosing the presence of MHE (Table 4).

Therefore, by taking into account the results obtained, the early diagnosis of minimal hepatic encephalopathy can be diagnosed *ex-vivo* by means of determining serum 3-nitrotyrosine.

For obtaining the serum of the patients and controls, the same protocol which has been followed in the present application can be followed, and the concentration of 3-nitrotyrosine is determined once the serum is obtained from the blood of the patients and controls.

In the example, the reversed-phase liquid chromatography (HPLC) technique has been used to determine 3-NT to separate it, and it has subsequently been detected by fluorescence as indicated above. Likewise, 3-NT could be detected with an electrochemical detector after separation by HPLC, or by means of other methods such as by identifying 3-NT by gas chromatography and mass spectrometry, for example, or even by means an ELISA assay using specific anti-3-nitrotyrosine antibodies. 3-NT can be determined by means of dot-blot using specific anti-3-NT antibodies.

Those patients whose 3-nitrotyrosine level is greater than the mean of the control subjects plus two standard deviations will be considered as MHE patients.

In the study performed 3-nitrotyrosine serum levels in control subjects reached a mean of 3.3 nM with a standard deviation of 2.2 nM. For this reason, the cut-off point for diagnosing the presence of MHE would be 7.7 nM. Those patients with a concentration of 3-nitrotyrosine greater than 7.7 nM would be considered as MHE patients and those with 7.7 nM or less would be considered as non-MHE patients.

This cut-off point could be slightly modified by increasing the number of studied individuals if the mean and/or the standard deviation of the control values were modified.

**Table 1. Liver disease etiology and analytical data**

| | Normal Range | Controls | Non-MHE patients | MHE patients |
|---|---|---|---|---|
| Total no. of | | 43 | 44 | 47 |
| individuals | | | | |
| Age | | 51±16 | 54±10 | 63±10 |
| Alcohol | | ---- | 44 | 47 |
| Ascites | | ---- | 6 | 10 |
| Child Pugh A/B/C | | ---- | 36/8/0 | 33/14/0 |
| MELD | | ---- | 8.4±3.0 | 9.3±2.5 |
| GOT (mU/ml) | (1-37) | 20±4 | 73±56^{c} | 83±58^{c} |
| GPT (mU/ml) | (1-41) | 18±6 | 77±24^{c} | 90±24^{C} |
| GGT (mU/ml) | (10-49) | 27±5 | 87±60^{c} | 106±64^{c} |
| Uric acid (mg/dl) | (2.5-7) | 4.0±1.0 | 6.2±2.0 | 5.7±2.3 |
| Creatinine(mg/dl) | (0.5-1.3) | 0.9±0.1 | 1.1±0.2 | 1.2±0.2 |
| Cholesterol (mg/dl) | (140-200) | 172±22 | 175±44 | 167±55 |
| Triglycerides (mg/dl) | (40-160) | 95±32 | 111±64 | 119±64 |
| Bilirubin (mg/dl) | (0.1-1) | 0.6±0.2 | 1.7±0.7^{c} | 2.3±0.6^{c} |
| Albumin (g/dl) | (3.5-5) | 4.4±0.2 | 3.7±0.6^{c} | 2.9±0.6^{c§} |
| Prothrombin time (sec) | | 13±1.3 | 24±4^{c} | 30±4^{c} |
| Fibrinogen (g/l) | (2-4) | 3.1±1.0 | 3.3±1.3 | 3.6±1.2 |
| Alkaline | (50-250) | 147±53 | 216±77^{b} | 314±9^{c}§ |
| phosphatase (mU/ml) | | | | |
| Erythrocytes | (4.2-6.1) | 4.6±0.4 | 4.3±0.7 | 3.4±0.6 |
| Leukocytes | (4.8-10.8) | 6.5±1.3 | 6.0±2.6 | 5.5±2.0 |
| Neutrophils (%) | (55-75) | 55±7 | 54±6 | 59±9 |
| Lymphocytes (%) | (17-45) | 35±6 | 29±10 | 27±9 |
| Monocytes (%) | (2-8) | 6.0±1.3 | 8.4±3.0^{b} | 10±2.6^{c} |
| Eosinophils (%) | (1-4) | 3.3±2.0 | 2.4±1.2 | 1.7±1.0 |
| Basophils (%) | (0.05-0.5) | 0.5±0.2 | 0.6±0.3 | 0.6±0.1 |

The values are given as mean±SD. The values significantly different from the controls are indicated with superscripts: ^{a} p<=0.05; ^{b} p<0.01; ^{c} p<0.001. The values significantly different in non-MHE and MHE patients are indicated as §, p<0.05.

| Table 2. Concentrations of serum amino acids | | | | |
|---|---|---|---|---|
| COMPOUND | Controls | Non-MHE patients p vs. control | MHE patients P vs. control | MHE patients P vs. non-MHE patients |
| **3-nitrotyrosine(n M)** | **3**.**3±0**.**35 (n=41**) | **6.0±0.7** (n= 42)** | **48±6*** (n=43)** | **p<0.0001** |
| Tyrosine (µM) | 65±6 (n=42) | 96±6** (n=43) | 104 ±8* (n=29) | NS |
| Phenylalanine (µM) | 50±4 (n=43) | 103 ±8*** (n=43) | 91±7*** (n=43) | NS |
| Glutamate (µM) | 79±7 (n=43) | 60±5* (n=40) | 48±4** (n=42) | NS |
| Glutamine (µM) | 406±28 (n=41) | 409±33 (n=43) | 516±35* (n=43) | p=0.027 |
| Aspartate (µM) | 4.4±0.5 (n=43) | 3.6±0.4 (n=43) | 4.7±0.6 (n=43) | NS |
| Asparagine (µM) | 36±3 (n=43) | 54±5** (n=43) | 66±6*** (n=43) | NS |
| **Citrulline (µM)** | **18±2 (n=41)** | **29 ±3* (n=43)** | **50±5*** (n=43)** | **p=0.002** |
| Alanine (µM) | 238±20 (n=41) | 336±44* (n=43) | 269±26 (n=43) | NS |
| Leucine (µM) | 72±4 (n=43) | 51 ±4*** (n=43) | 48±4*** (n=43) | NS |
| Isoleucine (µM) | 37 ±4 (n=43) | 49±4* (n=43) | 30±3 (n=43) | p=0.002 |
| Valine (µM) | 76±5 (n=41) | 53±3*** (n=43) | 46±4*** (n=43) | NS |
| **Methionine (µM)** | **19±2** | **44±3***** | **73±7***** | **p<0.0002** |
| | **(n=43)** | **(n=43)** | **(n=43)** | |
| Homocysteine (µM) | 4.6±0.5 (n=33) | 13±2*** (n=43) | 19±2*** (n=42) | p=0.017 |
| Homoserine (nM) | 2.3±0.3 (n=43) | 4.6±0.8** (n=43) | 4.2±0.5** (n=43) | NS |

The values are given as mean±SEM. The values significantly different from the controls are indicated with asterisks: p<0.05; ** p<0.01; *** p<0.001

| Table 3. Values of different parameters in controls and MHE or non-MHE patients | | | | |
|---|---|---|---|---|
| PARAMETER | Controls | Non-MHE patients p vs. control | MHE patients P vs. control | MHE patients P vs. non-MHE patients |
| Basal cGMP in lymphocytes (pmol/mg prot) | 0.23±0.03 (n=43) | 0.12±0.01** (n=44) | 0.06±0.01 *** (n=44) | p<0.001 |
| Activation of guanylate cyclase by nitric oxide in lymphocytes (times) | 14±1 (n=43) | 24±2*** (n=44) | 33±3*** (n=44) | p=0.05 |
| cGMP in plasma cGMP (nM) | 5.9±0.9 (n=43) | 15±2*** (n=44) | 21±2*** (n=44) | p=0.05 |
| Nitrates + Nitrites (µM) | 19±4 (n=43) | 25±5* (n=36) | 31±7** (n=34) | NS |
| The values are given as mean±SD. The values significantly different from the controls are indicated with asterisks: * p<0.05; ** p<0.01; *** p<0.001. | | | | |

**Table 4. Sensitivity, specificity and positive and negative predictive values of different compounds or combinations for the detection of MHE**

| PARAMETER | SENSITIVITY (%) | SPECIFICITY (%) | POSITIVE PREDICTIVE VALUE (%) | NEGATIVE PREDICTIVE VALUE (%) |
|---|---|---|---|---|
| **3-nitrotyrosine** | **82** | **71** | **75** | **79** |
| Methionine | 82 | 56 | 65 | 75 |
| Citrulline | 59 | 72 | 68 | 63 |
| Activation of guanylate cyclase | 46 | 66 | 56 | 56 |
| 3-nitrotyrosine + Methionine | 68 | 88 | 86 | 72 |
| 3-nitrotyrosine + Citrulline | 45 | 88 | 80 | 61 |
| Citrulline + Methionine | 48 | 91 | 84 | 63 |
| 3-nitrotyrosine + Methionine + Citrulline | 38 | 98 | 94 | 60 |

### References:

1. Amodio P, Montagnese S, Gatta A, et al. Characteristics of minimal hepatic encephalopathy. Metab Brain Dis. 2004; 19:253-267.
2. Ferenci P, Lockwood A, Mullen K, et al. Hepatic encephalopathy. Definition, Nomenclature, Diagnosis and Quantification: Final report of the working party at the 11th World Congress of Gastroenterology, Vienna, 1998. Hepatology 2002; 35: 716-721.
3. Montoliu, C., Piedrafita, B., Serra, M.A., del Olmo, J.A., Ferrandez, A., Rodrigo, J.M. and Felipo, V., (2007) Activation of soluble guanylate cyclase by nitric oxide in lymphocytes correlates with minimal hepatic encephalopathy in cirrhotic patients. J Mol Med. 85(3):233-241.
4. Montoliu, C., Piedrafita, B., Serra, M.A., del Olmo, JA, Urios, A., Rodrigo, J.M. and Felipo, V. (2009) IL-6 and IL-18 in blood may discriminate cirrhotic patients with and without minimal hepatic encephalopathy. J Clin Gastroenterol 43(3):272-279.
5. Romero-Gómez M, Boza F, García Valdecasas MS, et al. Subclinical hepatic encephalopathy predicts the development of overt hepatic encephalopathy. Am J Gastroenterol 2001;96:2718-2723.
6. Das A, Dhiman RK, Saraswat VA, et al. Prevalence and natural history of subclinical hepatic encephalopathy in cirrhosis. J Gastroenterol Hepatol 2001;16:531-535.
7. Groeneweg M, Quero JC, De Bruijn I, et al. Subclinical hepatic encephalopathy impairs daily functioning. Hepatology 1998;28:45-49.
8. Wein C, Koch H, Popp B, et al. Minimal hepatic encephalopathy impairs fitness to drive. Hepatology 2004;39:739-745.
9. Bajaj JS, Saeian K, Schubert CM, et al. Minimal hepatic encephalopathy is associated with motor vehicle crashes: the reality beyond the driving test. Hepatology. 2009;50(4):1175-1183
10. Romero-Gómez M, Boza F, Garda Valdecasas MS, et al. Subclinical hepatic encephalopathy predicts the development of overt hepatic encephalopathy. Am J Gastroenterol 2001;96:2718-2723.
11. Romero-Gómez M, Grande L, Camacho I. Prognostic value of altered oral glutamine challenge in patients with minimal hepatic encephalopathy. Hepatology. 2004;39(4):939-943.
12. Hernandez-Avila, Carlos A. and Ortega-Soto, Hector A. "Marcadores diagnósticos y mecanismos neuroquimicos de la encefalopatía secundaria a la insuficiencia hepática". Salud mental, 2001 vol.24, No. 1, p.48-59.
13. Marco Senzolo et al. "Minimal hepatic encephalopathy diagnosed by neuropsychological and neurophysiological tools in patients with liver cirrhosis". Digestive Disease Week/105th Annual Meeting of the American-Gastroenterological-association, USA, May 16-20, 2004. Pub: Gastroenterology, Apr 2004, Nr.4, Suppl.2, vol.126, p.A729. ISSN 0016-5085.
14. Keiding, Susanne et al. "Brain Metabolism of 13N-Ammonia During Acute Hepatic Encephalopathy in Cirrhosis Measured by Positron Emission Tomography". Hepatology, Jan. 2006, vol.43, p.42-50.
15. Suarez, I. et al. "Induction of NOS and nitrotyrosine expression in the rat striatum following experimental hepatic encephalopathy". Metab Brain Dis. 2009, Sep. 24(3):395-408.
16. Boris Görg et al. "Inflammatory cytokines induce protein tyrosine nitration in rat astrocytes". Archives of Biochemistry and Biophysics. 2006. Vol.449, p.104-114. ISSN 0003-9861. Available online at www.sciencedirect.com 9 March 2006.
17. Rabbani, Naila and Thornalley, Paul J. "Assay of 3-Nitrotyrosine in Tissues and Body Fluids by Liquid Chromatography with Tandem Mass Spectrometric Detection". Methods in enzymology. Vol.440, 2008 Elsevier Inc. p. 337-359. ISSN 0076-6879.
18. Mauricio Augusto Bragagnolo Jr. et al. "Minimal hepatic encephalopathy detection by neuropsychological and neurophysiological methods and the role of ammonia for its diagnosis". Archives of Gastroenterology, vol.46, Jan/March 2009, p.43-49.
19. Erceg, S., Monfort, P., Hernández-Viadel, M., Rodrigo, R., Montoliu, C. and Felipo, V. Oral administration of sildenafil restores learning ability in rats with hyperammonemia and with portacaval shunt. Hepatology. 2005; 45, 2-10
20. Erceg, S., Monfort, P., Hernandez-Viadel, M., Llansola, M., Montoliu, C., and Felipo, V. Restoration of learning ability in hyperammonemic rats by increasing extracellular cGMP in brain. Brain Res. 2005; 1036, 115-121
21. Cauli, O., Rodrigo, R., Piedrafita, B., Boix, J. and Felipo, V. Inflammation and hepatic encephalopathy: ibuprofen restores learning ability in rats with porto-caval shunts. Hepatology 2007; 46, 514-519.
22. World Medical Organization. Declaration of Helsinki. BMJ 1996; 313:1448-1449.
23. Weissenborn K, Ennen JC, Schomerus H, Rückert N, Hecker H. Neuropsychological characterization of hepatic encephalopathy. J Hepatol 2001;34:768-773
24. Schomerus H, Weissenborn K, Hamster W, Rückert N, Hecker H PSE syndrome test manual. Swets Test Services, Frankfurt, 1999.
25. Kimura M. Metallothioneins of monocytes and lymphocytes. Methods Enzymol 1989; 205:291-302.
26. Corbalán, R., Miñana, MD, Del Olmo, JA, Serra, MA, Rodrigo JM and Felipo, V. Altered modulation of soluble guanylate cyclase in lymphocytes from patients with liver disease J. Mol Med 2002;80:117-123
27. Hubbard WC, Bickel C, Schleimer RP. Simultaneous quantitation of endogenous levels of cortisone and cortisol in human nasal and bronchoalveolar lavage fluids and plasma via gas chromatography-negative ion chemical ionization mass spectrometry. Anal Biochem. 1994;221 (1):109-17.

## Claims

1. An *ex-vivo* method for the early diagnosis of minimal hepatic encephalopathy (MHE) in patients with liver diseases, including cirrhosis, **characterized in that** it comprises the following steps:
a) obtaining serum or plasma obtained from the blood of patients and controls, and
b) determining whether or not the measurement of the concentration of 3-nitrotyrosine obtained in (a) is greater than a specific level.

2. The method according to claim 1, **characterized in that** the patients having a concentration of 3-nitrotyrosine equal to or greater than the concentration value of 3-nitrotyrosine in control subjects plus 2 times the standard deviation value are considered as MHE patients.

3. The method according to claim 1, **characterized in that** the patients having a concentration of 3-nitrotyrosine less than the concentration value of 3-nitrotyrosine in control subjects plus twice the standard deviation value are considered as non-MHE patients.

4. The method according to claims 1-3, **characterized in that** 3-nitrotyrosine is determined by reversed-phase liquid chromatography HPLC and is detected by fluorescence.

5. The method according to claims 1-4, **characterized in that** the patients with a concentration of 3-nitrotyrosine equal to or greater than 7.7 nM are considered as MHE patients.

6. The method according to claims 1-4, **characterized in that** the patients with a concentration of 3-nitrotyrosine less than 7.7 nM are considered as non-MHE patients.

7. The method according to claims 1-3, **characterized in that** 3-nitrotyrosine is determined by reversed-phase liquid chromatography HPLC and is detected with an electrochemical detector.

8. The method according to claims 1-3, **characterized in that** 3-nitrotyrosine is determined by gas chromatography and mass spectrometry.

9. The method according to claims 1-3, **characterized in that** 3-nitrotyrosine is determined by means of an ELISA assay.

10. The method according to claims 1-3, **characterized in that** 3-nitrotyrosine is determined by means of dot-blot using specific anti-3-nitrotyrosine antibodies.

11. Use of 3-nitrotyrosine as a specific diagnostic marker for determining the presence of MHE in liver cirrhosis patients.

## Patentansprüche

1. Ex-Vivo-Verfahren zur Früherkennung von minimaler hepatischer Enzephalopathie (MHE) bei Patienten mit Lebererkrankungen, darunter Zirrhose, **dadurch gekennzeichnet, dass** es die folgenden Schritte umfasst:
a) Gewinnung von Serum oder Plasma, gewonnen aus dem Blut von Patienten und einer Kontrollgruppe, und
b) Bestimmung, ob das in (a) gewonnene Maß der Konzentration von 3-Nitrotyrosin größer als ein bestimmter Spiegel ist oder nicht.

2. Verfahren nach Anspruch 1, **dadurch gekennzeichnet, dass** die Patienten mit einer Konzentration von 3-Nitrotyrosin, gleich dem oder größer als der Konzentrationswert von 3-Nitrotyrosin bei den Kontrollteilnehmern plus 2-mal der Standardabweichungswert, als MHE Patienten angesehen werden.

3. Verfahren nach Anspruch 1, **dadurch gekennzeichnet, dass** die Patienten mit einer Konzentration von 3-Nitrotyrosin unter dem Konzentrationswert von 3-Nitrotyrosin bei den Kontrollteilnehmern plus doppelter Standardabweichungswert, als nicht- MHE-Patienten angesehen werden.

4. Verfahren nach den Ansprüchen 1-3, **dadurch gekennzeichnet, dass** 3-Nitrotyrosin durch Umkehrphasen-Flüssigchromatographie HPLC bestimmt wird und durch Fluoreszenz detektiert wird.

5. Verfahren nach den Ansprüchen 1-4, **dadurch gekennzeichnet, dass** die Patienten mit einer Konzentration von 3-Nitrotyrosin gleich oder größer als 7.7 nM als MHE Patienten angesehen werden.

6. Verfahren nach den Ansprüchen 1-4, **dadurch gekennzeichnet, dass** die Patienten mit einer Konzentration von 3-Nitrotyrosin unter 7.7 nM als nicht-MHE-Patienten angesehen werden.

7. Verfahren nach den Ansprüchen 1-3, **dadurch gekennzeichnet, dass** 3-Nitrotyrosin durch Umkehrphasen-Flüssigchromatographie HPLC bestimmt wird und mit einem elektrochemischem Detektor detektiert wird.

8. Verfahren nach den Ansprüchen 1-3, **dadurch gekennzeichnet, dass** 3-Nitrotyrosin durch Gaschromatographie und Massenspektrometrie bestimmt wird.

9. Verfahren nach den Ansprüchen 1-3, **dadurch gekennzeichnet, dass** 3-Nitrotyrosin mittels eines ELISA-Tests bestimmt wird.

10. Verfahren nach den Ansprüchen 1-3, **dadurch gekennzeichnet, dass** 3-Nitrotyrosin mittels Dot-Blot unter Verwendung von spezifischen Anti-3-Nitrotyrosin Antikörpern, bestimmt wird.

11. Verwendung von 3-Nitrotyrosin als spezifischer diagnostischer Marker für die Bestimmung der Anwesenheit von MHE bei Leberzirrhose-Patienten.

## Revendications

1. Procédé *ex-vivo* pour le diagnostic précoce de l'encéphalopathie hépatique minime (EHM) chez des patients atteints de maladies du foie, y compris la cirrhose, **caractérisé en ce qu'**il comprend les étapes suivantes :
a) obtenir du sérum ou du plasma obtenu à partir du sang de patients et de contrôles, et
b) déterminer si oui ou non la mesure de la concentration de 3-nitrotyrosine obtenue dans (a) est supérieure à un niveau spécifique.

2. Procédé selon la revendication 1, **caractérisé en ce que** les patients ayant une concentration de 3-nitrotyrosine égale ou supérieure à la valeur de concentration de 3-nitrotyrosine chez des sujets de contrôle plus 2 fois la valeur de déviation standard sont considérés comme des patients EHM.

3. Procédé selon la revendication 1, **caractérisé en ce que** les patients ayant une concentration de 3-nitrotyrosine inférieure à la valeur de concentration de 3-nitrotyrosine chez des sujets de contrôle plus deux fois la valeur de déviation standard sont considérés comme des patients non-EHM.

4. Procédé selon les revendications 1-3, **caractérisé en ce que** de la 3-nitrotyrosine est déterminée par chromatographie liquide en phase inverse HPLC et elle est détectée par fluorescence.

5. Procédé selon les revendications 1-4, **caractérisé en ce que** les patients avec une concentration de 3-nitrotyrosine égale ou supérieure à 7,7 nM sont considérés comme des patients EHM.

6. Procédé selon les revendications 1-4, **caractérisé en ce que** les patients avec une concentration de 3-nitrotyrosine inférieure à 7,7 nM sont considérés comme des patients non-EHM.

7. Procédé selon les revendications 1-3, **caractérisé en ce que** de la 3-nitrotyrosine est déterminée par chromatographie liquide en phase inverse HPLC et elle est détectée avec un détecteur électrochimique.

8. Procédé selon les revendications 1-3, **caractérisé en ce que** de la 3-nitrotyrosine est déterminée par chromatographie en phase gazeuse et spectrométrie de masse.

9. Procédé selon les revendications 1-3, **caractérisé en ce que** de la 3-nitrotyrosine est déterminée au moyen d'un essai ELISA.

10. Procédé selon les revendications 1-3, **caractérisé en ce que** de la 3-nitrotyrosine est déterminée au moyen de dot-blot en utilisant des anticorps spécifiques anti-3-nitrotyrosine.

11. Utilisation de 3-nitrotyrosine comme un marqueur diagnostique spécifique pour déterminer la présence d'EHM chez des patients atteints de cirrhose du foie.
